(19) Europäisches Patentamt
European Patent Office
Office européen des brevets

(11) Publication number: 0 392 717 A1

# (12) EUROPEAN PATENT APPLICATION

(21) Application number: 90303509.5

(22) Date of filing: 02.04.90

(51) Int. Cl.$^5$: A61K 47/48, A61K 47/26, A61K 47/18

(30) Priority: 04.04.89 US 333154

(43) Date of publication of application: 17.10.90 Bulletin 90/42

(84) Designated Contracting States: AT BE CH DE DK ES FR GB GR IT LI LU NL SE

(71) Applicant: ELI LILLY AND COMPANY
Lilly Corporate Center
Indianapolis Indiana 46285(US)

(72) Inventor: Pikal, Michael Jon
540 Leisure Lane
Greenwood, Indiana 46142(US)
Inventor: Roy, Michael Larry
4329 Abby Creek Lane
Indianapolis, Indiana 46205(US)

(74) Representative: Tapping, Kenneth George et al
Erl Wood Manor
Windlesham Surrey, GU20 6PH(GB)

## (54) Improvements in and relating to pharmaceutical formulations.

(57) Parenteral pharmaceutical formulations containing an immunoglobulin conjugate, glycine and mannitol, said formulations being stabilized against aggregation.

EP 0 392 717 A1

## IMPROVEMENTS IN AND RELATING TO PHARMACEUTICAL FORMULATIONS

The present invention relates to immunoglobulin conjugates and more particularly to pharmaceutical formulations containing said conjugates.

The alkaloids that are obtainable from *Vinca rosea* represent one of the most productive areas of chemistry for antineoplastic agents. Initially, only some of the alkaloids which were obtainable from the leaves of the plant by extraction were found to be active. These active antineoplastic alkaloids obtained directly from the leaves of the vinca plant include vinblastine (vincaleukoblastine or VLB), vincristine (leurocristine), leurosine (vinleurosine), leurosidine (vinrosidine), leuroformine (formylleurosine) and deoxy-VLB "A" and "B" (4′-deoxy-VLB and 4′-deoxyleurosidine). Other less abundant antineoplastic alkaloids have also been found. In addition to the native alkaloids, chemical modification of the indole-dihydroindole alkaloids obtained from *Vinca rosea* has created a wide variety of derivatives particularly with respect to chemical modification at positions C-3, C-4′ and C-4 of the molecule.

Further, monoclonal antibodies which define tumor-associated antigens have been shown to be effective vehicles for site-directed therapy by virture of covalent conjugation of these immunoglobulins with various oncolytic drugs. Given the interest in vinca alkaloids and chemical derivatives thereof and the potential of antibody conjugation thereto for site-directed chemotherapy, VLB, vincristine and other antineoplastic agents have been linked to immunoglobulins or other proteins. For example, U.K. Patent Application 2,090,837A discloses immunoglobulin conjugants covalently linked to a vinca moiety by amide formation. U.S. Patents 3,392,173 and 3,387,001 disclose C-4 esters of VLB, vincristine, leurosidine, and the like. Among these is a chloroacetyl ester, which derivative was employed to conjugate with a protein (see European Patent Application 124,502). Teale *et al.*, *Brit. J. Clin. Pharm.*, 4 169 (1977) disclose the conjugation of the vinca alkaloid to albumin by a Mannich reaction using an amine group in the protein (BSA), formaldehyde and vinblastine. Johnson *et al.*, *Brit. J. Can.*, 44, 372 (1981), disclose the preparation of vindesine linked to anti-CEA immunoglobulin via an azide. Other such conjugations of vinca alkaloids or chemical derivatives thereof to immunoglobulins are described in detail in U.S. Patent 4,801,688.

In said patent is disclosed a series of immunoglobulin conjugates formed by the reaction of an antineoplastic indole-dihydroindole vinca alkaloid containing a hydrazide group attached at C-3 or C-4 with an oxidized glycoprotein containing aldehyde groups. As disclosed in said patent, these conjugates are preferably administered parenterally. However, a difficulty has been encountered in the preparation of the parenteral pharmaceutical formulations in that the lyophilized immunoglobulin conjugate product tends to aggregate during processing and upon storage. The present invention addresses this problem by providing parenteral formulations which are stabilized against aggregation of the immunoglobulin conjugate product.

The present invention is directed to a parenteral pharmaceutical formulation which is stabilized against aggregation containing an immunoglobulin conjugate of an oxidized glycoprotein and a vinca hydrazide of the formula:

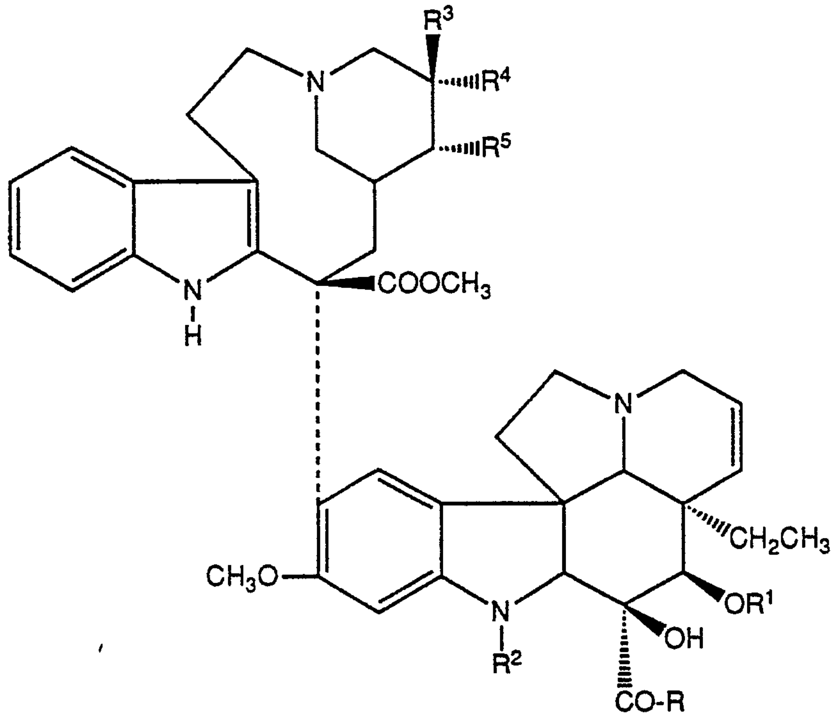

(I)

wherein $R^2$ is H, $CH_3$ or CHO; when $R^4$ and $R^5$ are taken singly, $R^5$ is H, and one of $R^3$ and $R^4$ is ethyl and the other is H or OH; when $R^4$ and $R^5$ are taken together with the carbons to which they are attached, they form an oxirane ring in which case $R^3$ is ethyl; R is $-NHNH_2$,

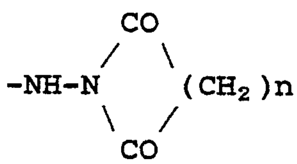

$-O(C_{1-3}$ alkyl), $NH_2$, $-NH(C_{1-3}$ alkyl), $-NH-CH_2CH_2-Y$, 1-pyrrolidinyl or 1-piperidinyl, wherein n is 2-4 and Y is Cl, $-OCH_3$ or $-SCH_3$; $R^1$ is H, $(C_{1-3}$ alkyl)-CO, chlorosubstituted $(C_{1-3}$ alkyl)-CO or $R^6$ wherein $R^6$ is $-COXCONHNH_2$ wherein X is $C_{1-4}$ straight chain alkylene, $C_{2-8}$ branched chain alkylene, $C_{2-4}$ alkenylene, $C_{3-4}$ alkynylene, $C_{3-6}$ cycloalkylene, phenylene, hydroxy-substituted $C_{1-4}$ alkylene, or a direct bond, except that R cannot be $NHNH_2$ when $R^1$ is $R^6$ and $R^1$ cannot be $R^6$ when R is $NHNH_2$, in admixture with aggregation stabilizing amounts of glycine and mannitol. Also provided by this invention is a method of reducing aggregation of an immunoglobulin conjugate of an oxidized glycoprotein and the vinca hydrazide of Formula I in a parenteral formulation by admixing said immunoglobulin conjugate with aggregation stabilizing amounts of glycine and mannitol.

As noted previously, the immunoglobulin conjugates utilized in the formulations and methods of the present invention are those disclosed and claimed in U.S. Patent 4,801,688 issued January 31, 1989 which is incorporated herein by reference. The conjugate is formed by the reaction of an oxidized glycoprotein containing one or more aldehyde groups with a vinca hydrazide. The hydrazide group can be either a C-3 carboxy hydrazide or a C-4 hydrazide-containing ester linked via a hydrocarbon chain. The hydrazides used in forming the conjugates employed in the present invention are prepared differently, depending on whether the hydrazide is attached at C-3 or C-4. The C-3 hydrazides are prepared by the procedure of U.S. Patent 4,203,898, which is incorporated herein by reference. When the hydrazide group is part of a C-4 chain, the 4-desacetyl starting materials are prepared by the procedure of U.S. Patent 3,392,173; J. Med. Chem., 22 391 (1979); U.S. Patent RE 30,560; U.S. Patent 4,357,334; U.S. Patent 4,203,898; and U.S. Patent 4,667,030, each of which are incorporated herein by reference.

The conjugates utilize an oxidized glycoprotein, preferably an immunoglobulin and, of that class, preferably a monoclonal antibody which is a gamma-globulin such as an IgG or an IgM. Immunoglobulin fragments containing carbohydrate, as in the parent immunoglobulin from which these fragments are

derived, can also be used to form these conjugates. The preferred class of glycoproteins, the immunoglobulins, are those which are reactive with or at least recognize antigens of the desired target cell. Particularly preferred are those glycoproteins which recognize antigens on the surface of the desired target cell. Immunoglobulin fragments referred to also as Fab, Fab′, F(ab′)$_2$ and IgM monomer derived from an antibody, by for example, proteolytic enzyme digestion or reductive alkylation, can also be used. A preferred immunoglobulin for use in the conjugate is the monoclonal antibody KS1/4.

The conjugation of the vinca hydrazides with oxidized glycoproteins is accomplished by standard methods known in the art. In general, a solution of the vinca in a water-miscible solvent such as dimethylformamide is added to a chilled buffered aqueous solution of the oxidized glycoprotein. Temperatures of about 0-8° C are preferred and a 0.1N sodium acetate buffer is normally employed. The reaction is best carried out in the dark and under an inert atmosphere. The reaction is normally complete in about 10-24 hours and the resulting conjugate may be purified by standard methods such as by chromatography over Sephadex. A particularly preferred conjugate is 4-desacetyl-3-carboxyhydrazide-monoclonal antibody KS1/4. Details concerning the preparation of all such conjugates may be found in U.S. Patent 4,801,688, supra.

The parenteral formulations of the present invention contain the immunoglobulin conjugate in association with aggregation stabilizing amounts of glycine and mannitol. By "aggregation stabilizing amounts" is meant those amounts of glycine and mannitol which produce a formulation which is stabilized against aggregation. By "stabilized against aggregation" is meant that less aggregation of the immunoglobulin conjugate occurs when said conjugate is formulated with aggregation stabilizing amounts of glycine and mannitol than when said conjugate is formulated in the absence of both glycine and mannitol. A preferred parenteral formulation of the immunoglobulin conjugate which is stabilized against aggregation is one containing said conjugate, glycine and mannitol in a 1:1:1 weight ratio, respectively. While this is a preferred formulation, the skilled artisan will readily appreciate that amounts of any one of the three constituents outside of this preferred ratio containing aggregation stabilizing amounts of glycine and mannitol may produce additional preferred parenteral formulations which are stabilized against aggregation. A formulation as disclosed herein containing an effective amount of an immunoglobulin conjugate is one containing from, for example, about 0.01 to 10 mg of the active constituent in terms of the vinca drug moiety.

The conjugates employed in the present formulations are prepared by the reaction of aldehyde-containing glycoproteins and a vinca hydrazide to form the cytotoxic hydrazone conjugates. However, the skilled artisan will readily appreciate that the particular type of chemical linkage employed to conjugate the indole-dihydroindole alkaloid (depicted as formula II in U.S. Patent 4,801,688) to the glycoprotein is irrelevant to the parenteral pharmaceutical formulations of the present invention. That is to say, the present invention will serve to stabilize against aggregation a formulation containing an immunoglobulin conjugate of an indole-dihydroindole alkaloid and a glycoprotein regardless of the chemical linkage employed between the two portions.

The preferred commercially available formulation is in the form of a lyophilized (i.e., freeze-dried) powder for reconstitution. Reconstitution of the formulation may be effected by the addition of a pharmaceutically acceptable vehicle therefor which includes water for injection, bacteriostatic water for injection, sterile water for injection and the like. The actual product preparation is conventional in the art including, for example, container selection, sterilization, filling and sealing as well as the inclusion of other additives such as antimicrobial agents, buffers, antioxidants, and the like. Further information relating to parenteral product preparations may be obtained from standard treatises such as Remington's Pharmaceutical Sciences, 17th Edition (1985), which is incorporated herein by reference.

The following examples are provided as a means of illustrating the present invention and are not to be construed as a limitation thereon.

## Example 1

### Preparation of 4-desacetyl VLB 3-carboxy-hydrazide-monoclonal antibody KS1/4 Conjugate

Using the procedure described in U.S. Patent 4,801,688, a solution was prepared by dissolving 200 mg of KS 1/4, a monoclonal antibody (moAb) capable of recognizing surface antigens of human adenocarcinoma cells, in 20 ml of a 0.1M sodium acetate buffer, pH 5.6 (29.3 g sodium acetate, 2.44 ml acetic acid plus sufficient sterilized water to make 4 L of buffer). The solution was stored at about 0° C overnight (about

10% of the protein had not dissolved). 685 mg of sodium meta-periodate were added in a single batch with rapid stirring.

The mixture was stirred for 21 minutes at about 0 °C in the dark and was then quenched by the addition of a 5-fold excess (for the total periodate) with 1.28 ml of a 12.5M solution of ethylene glycol in sterile water. The new mixture was stirred at 0 °C for 5 minutes in the dark and was then centrifuged to leave a clear supernatant and a white pellet. The supernatant was loaded onto a Sephadex G25 (medium mesh) gel column and the product eluted with the same sodium acetate buffer. The eluate was monitored with UV light at 280 nm. Any periodate was washed from the column and discarded. Concentration of the oxidized product was assessed in each eluate fraction at 279 nm; yield was 176 mg of oxidized MoAb in 39.9 ml of buffer (88% yield).

A solution of 274 mg of 4-desacetyl VLB 3-carboxhydrazide in DMF (5.6 ml of a 53.7 mg/ml solution) was prepared as described in U.S. Patent 4,801,688 and was added in dropwise fashion to the chilled buffered MoAb solution. The reaction vessel was flushed with nitrogen gas and then sealed. The reaction mixture was stirred in the cold and dark with magnetic stirring for 24 hours. The reaction vessel was then unsealed and the clear, pale yellow reaction mixture was centrifuged. The supernatant was chromatographed over Sephadex G25 gel preequilibrated with pH 7.4 phosphate buffered saline (0.01M $H_3PO_4$, 0.15M NaCl) which was also used as the eluant. The conjugate (formed by hydrazone formation between the 3-carboxhydrazide group and an aldehyde group in a carbohydrate on the MoAb) was eluted first, followed by unreacted 4-desacetyl VLB 3-carboxhydrazide. The yield of conjugate obtained was 146 mg (83% yield). The conjugate contained about 7.5 moles of 4-desacetyl-VLB-3-carboxhydrazone per mole of KS 1/4 MoAb.

In order to illustrate the aggregation stabilizing effect of the present invention on parenteral formulations of immunoglobulin conjugates, the following evaluation was conducted.

## Example 2

Formulations containing the conjugate of Example 1, glycine and mannitol were prepared in the weight ratios shown in Table I and were placed in parenteral vials and lyophilized. The formulations were maintained in storage for two months at 25 °C and were assayed at day 30 and day 60 for soluble aggregate formation of the conjugate by size exclusion chromatography. An initial assay was made following lyophilization (Day 0 as depicted in Table I). The results of these evaluations are shown in Table I.

Table I

| Formulation[a,b] | Percent Aggregation at Day Shown[c] | | |
|---|---|---|---|
| | 0 | 30 | 60 |
| 1:0:0 | 17 | 25.5 | NT[d] |
| 1:0:1 | 14.4 | 15.6 | 16.2 |
| 1:1:1 | 12.6 | 13.4 | 13.0 |

[a]Weight ratio of the conjugate of Example 1:glycine:mannitol, respectively
[b]All formulations contained 10 mM phosphate buffer
[c]Percent soluble aggregate formation as determined by size exclusion chromatography
[d]Not tested

As can be clearly seen from the data shown in Table I, the formulation containing the conjugate of Example 1, glycine and mannitol in a weight ratio of 1:1:1, respectively exhibited less aggregation at days 30 and 60 than the formulation in which glycine was lacking and exhibited less aggregation at day 30 than the formulation lacking both glycine and mannitol.

In order to show the aggregation stabilizing effect of the present invention on formulations maintained under conditions simulating those encountered during production, the following evaluation was conducted.

Example 3

Formulations containing the conjugate of Example 1, glycine and mannitol in the weight ratios shown in Table II (as well as phosphate buffer and NaCl in the amounts shown) were prepared and maintained at 5° C for 24 hours and then lyophilized. Following reconstitution, the formulations were visually inspected and assayed for soluble aggregate formation of the conjugate by size exclusion chromatography. The results of these evaluations are shown in Table II.

Table II

| Formulation[a] | Buffer[b] | NaCl[c] | Appearance | Percent Aggregation[d] |
|---|---|---|---|---|
| 1:0:0 | 10 | 0 | Precipitate | 12.9 |
| 1:0:0 | 50 | 0 | Precipitate | 11.8 |
| 1:0:0 | 10 | 3 | Precipitate | 10.9 |
| 1:1:1 | 10 | 0 | Clear | 7.8 |
| 1:1:1 | 50 | 0 | Clear | 9.2 |
| 1:1:1 | 10 | 3 | Clear | 7.4 |

[a]Weight ratio of the conjugate of Example 1:glycine:mannitol, respectively.
[b]Concentration of phosphate buffer (mM) present in formulation
[c]Concentration expressed in mg/ml
[d]Percent soluble aggregate formation as determined by size exclusion chromatography

**Claims**

1. A parenteral pharmaceutical formulation which is stabilized against aggregation comprising an immunoglobulin conjugate of an oxidized glycoprotein and a vinca hydrazide of the formula:

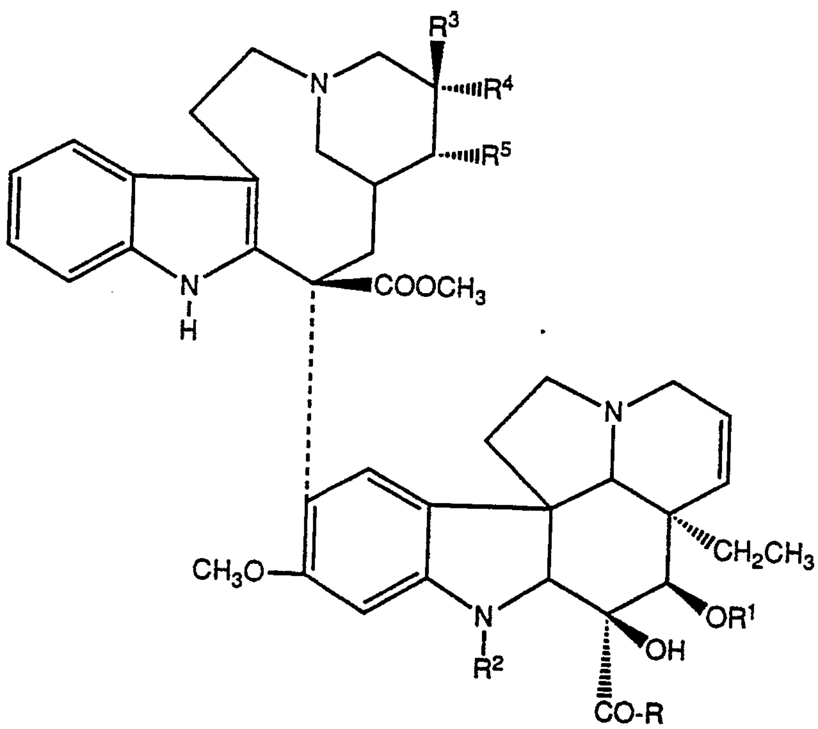

wherein $R^2$ is H, $CH_3$ or CHO; when $R^4$ and $R^5$ are taken singly, $R^5$ is H, and one of $R^4$ and $R^5$ is ethyl and the other is H or OH; when $R^4$ and $R^5$ are taken together with the carbons to which they are attached, they form an oxirane ring in which case $R^3$ is ethyl; R is $-NHNH_2$,

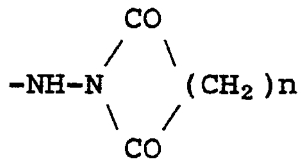

$-O(C_{1-3}$ alkyl), $NH_2$, $-N(C_{1-3}$ alkyl), $-NH-CH_2CH_2-Y$, 1-pyrrolidinyl or 1-piperidinyl, wherein n is 2-4 and Y is Cl, $-OCH_3$ or $-SCH_3$; $R^1$ is H, ($C_{1-3}$ alkyi)-CO, chloro-substituted ($C_{1-3}$ alkyl)-CO or $R^6$ wherein $R^6$ is $-COXCONHNH_2$ wherein X is $C_{1-4}$ straight chain alkylene, $C_{2-8}$ branched chain alkylene, $C_{2-4}$ alkenylene, $C_{3-4}$ alkynylene, $C_{3-6}$ cycloalkylene, phenylene, hydroxy-substituted $C_{1-4}$ alkylene, or a direct bond, except that R cannot be $NHNH_2$ when $R^1$ is $R^6$ and $R^1$ cannot be $R^6$ when R is $NHNH_2$, in admixture with aggregation stabilizing amounts of glycine and mannitol.

2. The formulation of Claim 1 in which the conjugate is 4-desacetyl VLB 3-carboxyhydrazide-monoclonal antibody KS 1/4.

3. The formulation of Claim 2 in which the conjugate, glycine and mannitol are present in a 1:1:1 weight ratio.

4. The formulation of any one of Claims 1, 2, or 3 as a lyophilized powder.

5. The formulation of any one of Claims 1, 2, 3, or 4 additionally containing a pharmaceutically acceptable vehicle.

6. A method of reducing aggregation of a parenteral pharmaceutical formulation containing an immunoglobulin conjugate of an oxidized glycoprotein and a vinca hydrazide of the formula:

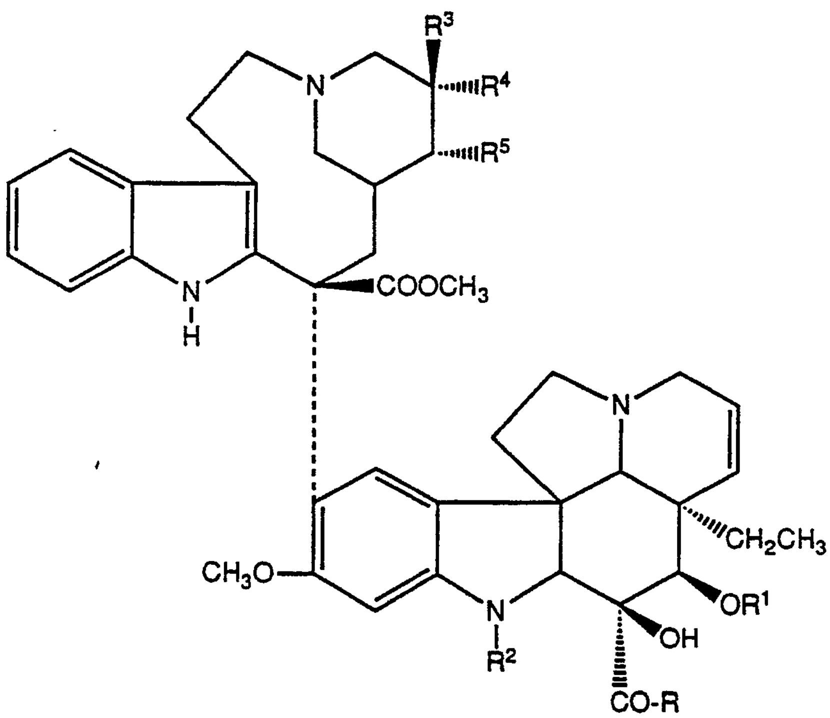

wherein $R^2$ is H, $CH_3$ or CHO; when $R^4$ and $R^5$ are taken singly, $R^5$ is H, and one of $R^3$ and $R^4$ is ethyl and the other is H or OH; when $R^4$ and $R^5$ are taken together with the carbons to which they are attached, they form an oxirane ring in which case $R^3$ is ethyl; R is $-NHNH_2$,

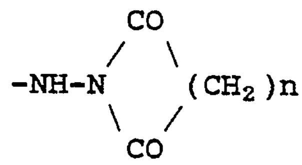

-O($C_{1-3}$ alkyl), $NH_2$, -NH($C_{1-3}$ alkyl), -NH-$CH_2CH_2$-Y, 1-pyrrolidinyl or 1-piperidinyl, wherein n is 2-4 and Y is Cl, -$OCH_3$ or -$SCH_3$; $R^1$ is H, ($C_{1-3}$ alkyl)-CO, chloro-substituted ($C_{1-3}$ alkyl)-CO or $R^6$ wherein $R^6$ is -COXCONHNH$_2$ wherein X is $C_{1-4}$ straight chain alkylene, $C_{2-8}$ branched chain alkylene $C_{2-4}$ alkenylene, $C_{3-4}$ alkynylene $C_{3-6}$ cycloalkylene, phenylene, hydroxy-substituted $C_{1-4}$ alkylene, or a direct bond, except that R cannot be $NHNH_2$ when $R^1$ is $R^6$ and $R^1$ cannot be $R^6$ when R is $NHNH_2$ comprising admixing said conjugate with aggregation stabilizing amounts of glycine and mannitol.

7. The method of Claim 6 wherein the conjugate is 4-desacetyl VLB 3-carboxyhydrazide-monoclonal antibody KS1/4.

8. The method of Claim 7 in which the conjugate, glycine and mannitol are present in a 1:1:1 weight ratio.

9. The method of any one of Claims 6, 7, or 8 wherein said formulation is in the form of a lyophilized powder.

10. The method of any one of Claims 6, 7, 8, or 9 wherein the formulation additionally contains a pharmaceutically acceptable vehicle.

11. A process for preparing a parenteral pharmaceutical formulation which comprises admixing an immunoconjugate as defined in Claim 1 with glycine and mannitol.

European Patent Office

# PARTIAL EUROPEAN SEARCH REPORT

which under Rule 45 of the European Patent Convention shall be considered, for the purposes of subsequent proceedings, as the European search report

Application number: EP 90 30 3509

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,Y | EP-A-0 326 322 (ELI LILLY AND CO.)<br>* Claims 1,6 *<br>-- | 1-11 | A 61 K 47/48<br>A 61 K 47/26<br>A 61 K 47/18 |
| D,Y | EP-A-0 247 792 (ELI LILLY AND CO.)<br>* Claims *<br>& US-A-4 801 688<br>-- | 1-11 | |
| Y | WO-A-89 09 614 (GENENTECH, INC.)<br>* Claims; page 11, lines 5-7 *<br>-- | 1-11 | |
| A | BE-A- 732 141 (Mds L. OLIVIER et al.)<br>* Claims *<br>-------- | | |

TECHNICAL FIELDS SEARCHED (Int. Cl.4): A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:

Claims searched incompletely: 1-5

Claims not searched:

Reason for the limitation of the search:

In the first claim on the 7th line and the sixth claim mentions a vinca hydrazide of the formula...
Nevertheless the combinations of R and $R_1$ in the formula also allows many combinations which are not hydrazides. For this reason the search has been performed in the light of the examples and with the promis that one of R or $R_1$ is a hydrazide or hydrazidederivatives as described in the claims 1 or 6.

| Place of search | Date of completion of the search | Examiner |
|---|---|---|
| The Hague | 23-07-1990 | BERTE |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document